# EUROPEAN PATENT SPECIFICATION

(11) **EP 1 590 312 B1**
(45) Date of publication and mention of the grant of the patent: **13.04.2011**
(21) Application number: 04706453.0
(22) Date of filing: 29.01.2004
(51) Int. Cl.: C07C 45/29, C07C 45/30

(54) **BROMINE FREE TEMPO BASED CATALYST SYSTEM FOR OXIDATION OF PRIMARY AND SECONDARY ALCOHOLS USING NAOCI AS AN OXIDANT.**
BROMFREIES KATALYSATORSYSTEM AUFTEMPO-BASIS ZUR OXIDATION PRIMÄRER UND SEKUNDÄRER ALKOHOLE MIT NAOCI ALSOXIDATIONSMITTEL
SYSTEME DE CATALYSEUR TEMPO EXEMPT DE BROME DESTINE A L'OXYDATION D'ALCOOLS PRIMAIRES ET SECONDAIRES, FAISANT APPEL A NAOCL EN TANT QU'OXYDANT

(30) Priority: 30.01.2003 US 443749 P
(43) Date of publication of application: 02.11.2005
(73) Proprietor: NutraSweet Property Holdings, Inc., Chicago IL 60654 (US)
(72) Inventor: TANIELYAN, Setrak, K., Maplewood, NJ 07040 (US); AUGUSTINE, Robert, L., Livingston, NJ 07039 (US); PRAKASH, Indra, Hoffman Estates, IL 60195 (US); FURLONG, Kenneth, E., Evans, GA 30809 (US); SCHERM, Robert, C., Evans, GA 30809 (US); JACKSON, Handley, E., North Augusta, SC 29841 (US)
(74) Representative: Ebner von Eschenbach, Jennifer
(86) International application number: PCT/US2004/002475
(87) International publication number: WO 2004/067484

(56) References cited:
- US-A- 5 856 584
- US-B1- 6 451 943
- US-B1- 6 518 419
- MA ET AL: 'Organic Oxoammonium Salts. 3.1 A New Convenient Method for the Oxidation of Alcohols to Aldehydes and Ketones' JOURNAL OF ORGANIC CHEMISTRY vol. 56, 1991, pages 6110 - 6114, XP000229776

## Description

### FIELD OF THE INVENTION

The present invention relates to bromine free catalyst system, which exhibits high activity and selectivity in the oxidation of alcohols to aldehydes or acids using NaOCl as an oxidant. More specifically, the invention relates to a catalyst system, which comprises a synergistic couple of 2,2,6,6,-tetramethylpiperidinyloxy based catalysts (hereinafter referred to as "TEMPO" or "TEMPO catalyst") and Na₂B₄O₇ co-catalyst (CC), which is more active and shows higher selectivity than the known TEMPO - NaBr system. Such a synergistic couple is particularly useful for, but not limited to, oxidation of primary aliphatic and aromatic alcohols. The system also permits carrying out the oxidation without the need for solvents.

### BACKGROUND OF THE INVENTION

The catalytic oxidation of alcohols selectively to carbonyl compounds is probably one of the most important transformations in the synthetic organic chemistry. A large number of oxidants have been reported in the literature and most of them are based on transition metal oxides such as chromium and manganese (S. Kirk-Othmer Mitchell, Enciclopedia of Chemical Technology, 4th ed., Wiley -Interscienc, New York, Vol.2, p 481, (1992); Hudlicky, M. "Oxidations in Organic Chemistry", ACS Monograph No.186 American Chemical Society Washington D.C. (1990); Sheldon R.A., Kochi J.K. Metal Catalized Oxidation of Organic Compounds. New York, Academic Press, 1981; Ley, S.V., Madin, A,. In comprehensive Organic Synthesis, Trost B., Fleming, I., Eds.; Pergamon Oxford, 1991; Vol 7, p251; Mijs, W.J., DeJonge, C.R.H.I. Organic Synthesis by Oxidation with Metal Compounds ; Plenum: New York, 1968). Since most of the oxidants and the products of their transformation are toxic species, their use creates serious problems concerning their handling and disposal. A serious drawback to the use of these reagents, for both cost and toxicity reasons, is the need to use them in large excess over the required reaction stoicheometry. The search for efficient, easily accessible catalysts and "clean" oxidants such as hydrogen peroxide, hydroperoxides or molecular oxygen for industrial applications is still a challenge (Dijksman, A., Arends I.W.C.E. and Sheldon R., Chem. Commun., 1999, 1591-1592; Marko I.E., P.R.Giles, Tsukazaki M., Brown S.M. and Urch C.J., Science, 19696, 274, 2044). A large number of transition metal complexes and oxidants have been reported to catalyze the selective oxidation of primary alcohols to aldehydes with varying levels of effectiveness such as RuCl₃,NaBrO₃ (Konemoto S., Tomoioka S., Oshima K.), Bull. Chem. Soc. Japan. 1986. V.59. N1, P.105), Bu₄NRuO₄-4-Methylmorpholine N-oxide (Griffith W.P., Ley S.V., Whitcombe G.P., White A.D)., Chem. Commun. 1987, N21, p.1625), H₂O₂ and tert-Butylhydroperoxide (t-BuOOH) (Y. Tsuji, T. Ohta, T. Ido et al.), J. Organometalic Chemistry, 270, 333 (1984), (T.M. Jiang, J.C. Hwang, H.O. Ho, C.Y. Chen), J. Chin. Chem. Soc., 35, 135, (1988). The methods described have only limited use since the overall yields are low and some of them require the application of precious metal complexes or expensive primary oxidants.

A particularly convenient procedure for the oxidation of primary and secondary alcohols is reported by Anelli and co-workers (J. Organic Chemistry, 1987, 52, 2559; J. Organic Chemistry, 1989, 54, 2970). The oxidation has been carried out in a two-phase system (CH₂Cl₂-water) utilizing the TEMPO as a catalyst and cheap and readily accessible NaOCl as an oxidant. The co-catalyst KBr enhances the reaction rate and the aqueous phase is buffered at pH 8.5-9.5 using NaHCO₃. The use of a quaternary ammonium salt as a phase transfer catalyst furthers the oxidation of alcohols to carboxylic acids. The same procedure was modified by using NaClO₂ as the oxidant in the presence of catalytic amounts of TEMPO and NaOCl. This led to the formation of the carboxylic acid as the main product (U.S. Patent No. 6,127,573).

Prakash et al. in U.S. Patent No. 5,856,584 report a similar procedure for oxidation of 3,3-Dimethyl-1-butanol. According to this procedure the 3,3-Dimethyl-1-butanol is oxidized to 3,3-Dimethylbutyraldehyde with NaOCl in a two-phase system using CH₂Cl₂ as a reaction solvent. The stable 2,2,6,6-tetramethyl-1-piperidinyloxy free radical and KBr are used as an efficient catalytic system to produce the desired aldehyde in 80% isolated yield.

In another development of the TEMPO mediated oxidation of primary alcohols to aldehydes, Sheldon and co-workers have used a polymeric version of the TEMPO catalyst PIPO, obtained by oxidation of the commercially available poly[(6-[1,1,3,3-tetramethylbutyl)amino]-s-triazine-2,4-diyl][(2,2,6,6-tetramethyl-4-piperidyl)imino] hexamethylene[(2,2,6,6-tetramethyl-4-piperidinyl)imino]], known also as Chimasorb 944, (in Chem.Commun., 2000, 271-272). The procedure produces aldehydes in high yields in solvent free conditions only for aromatic alcohols. The oxidation of primary alcohols leads to significant over-oxidation to carboxylic acid.

U.S. Patent No. 5,821,374 describes the use of N-chloro compounds such as N-chloro-4-toluenesulfonamide sodium salt (Chloramine T) or N-chloro-benzene sulfonamide sodium salt (Chloramine B) as an oxidant in the TEMPO catalyzed oxidation of primary alcohols to aldehydes. The major drawback to this method is the use of large amounts of solvents and the toxicity of the N-chlorinated aromatics used as oxidants.

U.S. Patent No. 6,335,464 describes a polymer supported TEMPO catalyst which, combined with a NaBr co-catalyst, was used to electrocatalytically oxidize primary alcohols to carboxylic acids using NaOCl as the oxidant. There is no report on the use of the procedure for selectively forming the corresponding aldehydes and the possibility for re-use of the catalyst.

Despite the extensive work reported in the area of the selective oxidation of primary alcohols there is still a continuous need for developing highly efficient and economical oxidation methods which do not require the use of organic solvents, can be carried out with environmentally friendly oxidants and do not require the use of bromine based co-catalysts. It is the object of the present invention to provide such an oxidation method.

### SUMMARY OF THE INVENTION

The process according to this invention comprises oxidizing primary or secondary alcohols with an oxidant in the presence of a catalyst of formula II or III and a co-catalyst.

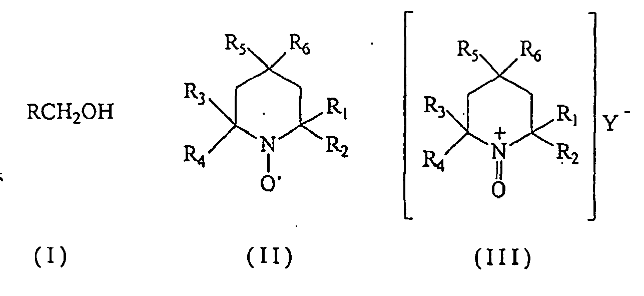

In Formulas (II) and (III), R₁, R₂, R₃ and R₄ independently are lower alkyl or substituted alkyl groups of the same or different structures. R₅ and R₆ are hydrogen, alkyl or lower alkoxy or one is hydrogen and the other is lower alkoxy, hydroxy, amino, alkyl or dialkylamino, alkylcarbonyloxy, alkylcarbonylamino, or R₅ and R₆ are ketal. The Y group is an anion.

The co-catalyst according to the invention is a Na₂B₄O₇ co-catalyst.

The TEMPO/co-catalyst promoted oxidation is described by the following reaction shown in Scheme 1. According to this, the oxidation takes place via a cascade mechanism in which a number of oxidizing species exist in a dynamic equilibrium. Formally, the hypochlorite anion oxidizes the co-catalyst (CC) to its oxidized form (CCO*), which in turn transfers the chain over to TEMPO oxoammonium salt. The "oxidized" form of TEMPO converts the primary alcohol to aldehyde in the last redox cycle. The reaction takes place at the pH of the bleach solution in the range of 8.6-9.5 in which the hypochlorite anion is relatively stable and at the same time the rate within the ClO⁻/Cl⁻ - CC/CCO* is high enough to sustain high overall rates of alcohol oxidation. To maintain the desired pH, a NaHCO₃ or K₂CO₃ could be effectively used. Since the desired aldehyde under the current oxidizing conditions can easily undergo further conversion to the corresponding acid derivative, the reaction temperature is kept at or below 0°C.

In one embodiment of the invention two or more catalysts each selected from the group consisting of 2,2,6,6,-tetramethylpiperidinyloxy based catalysts are included in said alcohol solution.

### DETAILED DESCRIPTION OF THE INVENTION

The process according to the invention comprises oxidizing primary or secondary 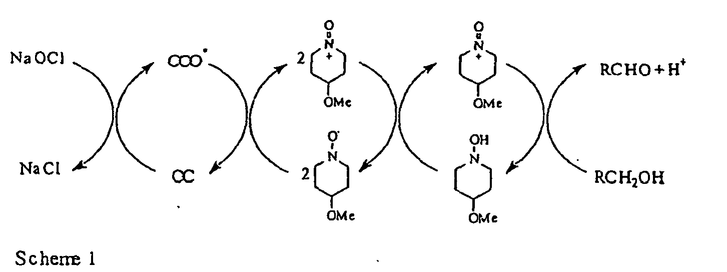 alcohols with an oxidant in the presence of a TEMPO based catalyst of formula II or III above and the co-catalyst.

The term primary or secondary alcohols as used in the present invention describes organic compounds having primary or secondary hydroxyl groups. The term lower alcohol as used herein refers to alcohols having 1 to 10 carbon atoms while the term higher alcohol as used herein refers to alcohols having 11 or more carbon atoms. Examples of primary and secondary alcohols thereof include alcohols such as methanol, ethanol, n- and isopropyl alcohol, n-, iso- and see-butyl alcohol, pentyl alcohol, hexyl alcohol, neopentyl alcohol, neohexyl alcohol, octyl alcohol, lauryl alcohol, tridecyl alcohol, myristyl alcohol, nonadecyl alcohol, eicosyl alcohol. Examples of unsaturated alcohols include allyl alcohol, crotyl alcohol and propargyl alcohol. Examples of aromatic alcohols include benzyl alcohol, phenyl ethanol, phenyl propanol and the like. The term TEMPO based catalyst as used herein refers to compounds of formula II or III above. Here, R₁, R₂, R₃ and R₄ independently are lower alkyl or substituted alkyl groups of the same or different structures. R₅ and R₆ are both hydrogens, alkyl or are lower alkoxy or one is hydrogen and the other is lower alkoxy, hydroxy, amino, alkyl or dialkylamino, alkylcarbonyloxy, alkylcarbonylamino, or can jointly be an oxygen or ketal. The Y⁻ group is an anion.. The Y⁻ group is an anion. The term "lower alkyl" means straight chain or branched saturated hydrocarbon groups with up to 8 carbon atoms, such as methyl, ethyl, n-propyl, isopropyl, isobutyl, pentyl, n-hexyl and the like. The term "lower alkoxy" means lower alkyl groups bonded via an oxygen atom, such as methoxy, ethoxy and the like. The term "lower alkylcarbonyloxy means lower alkylcarbonyl group bonded via an oxygen atom. The term "lower alkylcarbonyl" means lower alkyl groups bonded via carbonyl group and is represented by groups such as acetyl, propionyl and the like. The term "lower carbonylamino" means lower alkylcarbonyl group bonded via nitrogen atom such as acetylamino and the like.

Examples of such compounds include, but are not limited to 2,2,6,6,-tetramethylpiperidine N-oxyl (TEMPO) and the 4-substituted derivatives thereof including 4-methoxy-TEMPO, 4-ethoxy-TEMPO, 4-acetoxy-TEMPO, 4-acetamino-TEMPO, 4-hydroxy-TEMPO, 4-benzoyloxy-TEMPO, 4-amino -TEMPO, N, N-dimethylamino-TEMPO, 4- oxo-TEMPO and the polymeric versions of TEMPO such as poly [(6-[1,1,3,3-tetramethylbutyl)amino]-s-triazine-2, 4-diyl], [(2,2,6,6-tetramethyl-4-piperidyl)imino] hexamethylene[(2,2,6,6-tetramethyl-4-piperidinyl)imino]], known also as Chimasorb 944.

The term oxidant as used herein means compounds capable of either transferring active oxygen to the co-catalyst or directly oxidizing the reduced form of the TEMPO catalyst (see Scheme 1). Suitable oxidizing agents that can be employed include, but are not limited to chlorite, chlorate, bromate, hypochlorite, hypobromite, hydrogen peroxide, organic hydroperoxides, percarboxylic acids and the like. More particularly, sodium hypochlorite, potassium hypochlorite, calcium hypochlorite, sodium hypobromite, potassium hypobromite tert-butyl hydroperoxide, trichloroisocyanuric acid, peracetic acid, performic acid, trichloroperacetic acid or trifluoroperacetic acid are used. Commercial scale bleach is especially preferred. Bleach can be use as is or can be modified by any inorganic salt such as chloride, sulfate, carbonate of sodium, potassium, magnesium, bases such as sodium or potassium carbonates, bicarbonates, or acids such as acetic, hydrochloric, or sulfuric to improve the ionic strength and modify the pH. In particular a bleach solution is added to the solution the addition time being between completion addition at one time and 10 hours and post addition reaction is continued for an additional 0 to 10 hours, and preferably 0 to 30 hours.

The presence of solvents in the process of the invention is not critical. The reaction can be carried out in neat alcohol with the same efficiency and the same level of selectivity. In the instances when the alcohol to be oxidized is solid at the reaction temperatures employed, any conventional non-polar, aprotic solvent may be used. Particularly preferred solvents include but are not limited to methylene chloride, chloroform, ethyl acetate, butyl acetate, acetonitrile, tetrahydrofuran, toluene, acetone, diethyl ether, methyl tert-butyl ether, pentane, hexane or a mixture of such solvents. Especially preferred solvents are heptane, toluene and ethyl acetate.

One or more solvents may be added.

Inorganic salt addition to the reaction system increases the ionic strength of the system and lowers the freezing point of the aqueous buffer solution. Such depression in the freezing point of the reaction mixture is needed when the oxidation reaction is carried out in solvent-free conditions. Suitable inorganic salts are selected from the group including, but not limited to, sodium chloride and potassium chloride.

The accumulation of small amounts of organic acid and HCl may drive the pH of the reaction solution to lower values and will cause reduction in the reaction rate. Therefore, to achieve maximum rate and high product selectivity, the oxidation reaction is preferably carried out in presence of an aqueous buffer or base to intercept the acidic by-products formed. Suitable buffering agents and base compounds are selected from the group including but not limited to NaHCO₃, KHCO₃, Na₂CO₃, K₂CO₃, K₂PO₄, Na₂HPO₄, NaH₂PO₄, K₂HPO₄, KH₂PO₄, NaOAc. They are used in amounts sufficient to maintain a pH of about 4-12 and most preferably 8.6-9.5.

The reaction can be carried out across a range of temperatures. The temperature at which the oxidation of the present invention is carried out is an important variable affecting aldehyde selectivity. The preferred reaction temperature is in the range from -10°C - 50°C or -15°C to 30°C, most preferably in the range from 0°C to 10°C. The process of the invention can be carried out in any conventional batch, semi-batch or continuous flow reactor capable of bringing the two immiscible phases is sufficient contact and at the same time capable of maintaining the reaction temperature within the desired range.

The process may comprise the additional step of purification of the crude aldehyde or ketone via distillation, fractional distillation other batch or continuous or a thin film evaporator. The 2,2,6,6-tetramethylpiperidinyl based catalyst is preferably present in an amount of 0.001 - 10 mol% of the substrate alcohol whilst the ratio between the oxidant and the alcohol is in the range of from 1: 0.8 to 1 1.5.

In accordance with one embodiment of the present invention, the oxidation is carried out as follows:
1. Preparing an aqueous solution of NaHCO₃ and Na₂BaO₇.
2. Adding a solution of MeO-TEMPO in an alcohol substrate of Formula I
3. Bringing the pH of the biphasic system into the range 8.6-9.5 using a dilute solution of HCl or CH₃COOH
4. Cooling the stirred suspension to about 0-5°C
5. Starting the metered addition of concentrated bleach solution maintaining the reaction temperature at the desired setting to oxidize the compound of Formula I and
6. Post-addition stirring of the reaction to full conversion of the substrate.

In accordance with another embodiment of the present invention the oxidation is carried out in the presence of a reaction solvent:
1. Preparing an aqueous solution of NaHCO₃ and Na₂B₄O₇
2. Adding a solution of MeO-TEMPO and alcohol substrate of Formula I in appropriate reaction solvent
3. Bringing the pH of the biphasic system into the range 8.6-9.5 using a dilute solution of HCl or CH₃COOH
4. Cooling the stirred suspension to about 0-5°C
5. Starting the metered addition of concentrated bleach solution maintaining the reaction temperature at the desired setting to oxidize the compound of Formula I and
6. Post-addition stirring of the reaction to full conversion of the substrate.

Also disclosed the oxidation is carried out in presence of a ZrO (acetate)₂ co-catalyst and comprises the following steps:
1. Preparing an aqueous solution of NaHCO₃ and ZrO (acetate)₂
2. Adding a solution of MeO-TEMPO and alcohol substrate of Formula I
3. Bringing the pH of the biphasic system into the range 8.6-9.5 using a dilute solution of HCl or CH₃COOH
4. Cooling the stirred suspension to about 0-5°C
5. Starting the metered addition of concentrated bleach solution maintaining the reaction temperature at the desired setting to oxidize the compound of Formula I and
6. Post-addition stirring of the reaction to full conversion of the substrate

In another embodiment of the present invention the oxidation is carried out with pre-activated bleach solution and could be described as:
1. Preparing an aqueous solution of NaHCO₃ and Na₂B₄O₇
2. Adding a solution of MeO-TEMPO and alcohol substrate of Formula I in an appropriate reaction solvent
3. Bringing the pH of the biphasic system into the range 8.6-9.5 using a dilute solution of HCl or CH₃COOH
4. Cooling the stirred suspension to about 0-5°C
5. Starting the metered addition of concentrated bleach solution, the pH of which has been reduced by using dilute solution of HCl or CH₃COOH and
6. Post-addition stirring of the reaction to full conversion of the substrate

In the processes of the present invention, the solvent, if used, is preferably selected from the group of aprotic inert solvents such as methylene chloride, chloroform, ethyl acetate, butyl acetate, acetonitrile, tetrahydrofuran, toluene, acetone, diethyl ether, methyl *tert*-butyl ether, pentane, hexane, heptane and the mixture of the said solvents. Especially preferred solvents are heptane, toluene and ethyl acetate or a mixture of said solvents.

In the inventive processes, the MeO-TEMPO catalyst preferably is used in a concentration of 0.001 -10.0 % mol, more preferably about 0.1-1% mol. The co-catalyst preferably is used in a concentration of 0.002 -20.0% mol, more preferably about 0.2-2 mole percent. If the bleach solution comprises sodium hypochlorite, the sodium hypochlorite preferably is used in about 0.8-1.5 equivalents, more preferably about 1-1.2 equivalents, relative to the alcohol substrate.

Once the reaction is completed, the crude 3,3-dimethylbutyraldehyde is isolated by phase split or by extraction with organic solvent. The solvent used in extraction can be selected from the group of aprotic inert solvents such as methylene chloride, chloroform, ethyl acetate, butyl acetate, methyl acetate, toluene, diethyl ether, methyl *tert*-butyl ether, pentane, hexane, heptane. Excess solvent may be recycled after isolation of the desired aldehyde. Especially preferred solvents are toluene and ethyl acetate. The crude 3,3-dimethylbutyraldehyde can be recovered in several ways, including distillation, fractional distillation, either batch or continuous, or use of a thin-film evaporator to concentrate the 3,3-dimethylbutyraldehyde. The crude 3,3-dimethylbutyraldehyde can be purified as described in US patent No. 5,905,175 or by distillation, fractional distillation, either batch or continuous, or use of a thin-film evaporator. A preferred purification step involves distillation at 100-106°C and atmospheric pressure to obtain purified 3,3-dimethylbutyraldehyde.

The following examples are given to illustrate the scope of the present invention. The examples are given for illustrative purposes only; the invention embodied herein should not be limited thereto.

### EXAMPLE I-Comparative Example

Example I represent a reference oxidation reaction under the conditions analogous to the one (reported in J.Organic Chemistry, 1987, 52, 2559 and J. Organic Chemistry, 1989, 54, 2970), also known as Anelli protocol.

820 mg of 3,3-Dimethyl-1-butanol (8 mmol) and 14.9 mg MeO-TEMPO (0.08 mmol) are dissolved in toluene (20 cc) in a jacketed glass reaction flask, equipped with a thermocouple, an addition port, a Teflon coated magnetic stir bar and a pH probe. Potassium bromide (45.25 mg, 0.4 mmol) and 1310mg of NaHCO₃ are dissolved in water (21.6 cc) and the aqueous phase is added to the stirred organic fraction at 1000 RPM in the reaction flask. The stirred suspension is cooled to 0°C and 5.4 g of 12.3% aqueous NaOCl (8.92 mmol) are added via a gas-tight syringe over 5 minutes. The reaction mixture is aged for an additional 30 min and the organic layer sampled for GC assay. The yield of 3,3-dimethylbutyraldehyde is 77% at 30 min and 91% at 60 min reaction time.

The following are the calculated amounts of MeO-TEMPO catalyst, co-catalyst and buffering reagents needed for the manufacture of 1 Kg 3,3-dimethylbutyraldehyde.

**Table 1**

| 1 | 2 | 3 |
|---|---|---|
| Component | 8 mmol scale | 1 kg scale |
| | G (cc) | Kg (L) |
| 3,3-dimethyl-1-butanol | 0.817 | 1.00 |
| Toluene | (20) | (24.5) |
| MeO-TEMPO | 0.0149 | 0.018 |
| H₂O | (22) | (26.9) |
| KBr | 0.0475 | 0.300 |
| NaHCO₃ | 1.31 | 0.160 |
| NaOCl | 5.1 | 6.24 |

The data from Table 1 show the serious deficiencies of the known procedure for TEMPO based bleach oxidation of alcohols, which makes it economically not feasible for practical applications. These include the recommended use of large amounts of chlorinated or other solvents, large amounts of MeO-TEMPO catalyst per unit amount substrate, excessive volumes of aqueous buffer solution and the use of potassium bromide co-catalyst in concentration of at least 5% by weight of the alcohol substrate.

### EXAMPLE II-Comparative Example

Example II represents a scaled up synthesis performed in the presence of KBr co-catalyst and NaHCO₃ buffer in the absence of a reaction solvent. Number of improvements are made to allow the use of neat alcohol, such as reduced amount of buffer, sharply reduced amount of KBr, slow addition of the bleach solution and continuous maintenance of the pH in the stage of the bleach addition and further during the post addition reaction. Example II is the closest approximation to the Anelli conditions and although it is a qualitatively new level on its own compared to the prior art, is considered here for reference purposes only.

16.9 g of 3,3-Dimethyl-1-butanol (117.3 mmol) and 0.0765 g MeO-TEMPO (0.411 mmol) are charged in a jacketed glass reaction flask, equipped with a thermocouple, an addition port, a Teflon coated magnetic stir bar and a pH probe. Potassium bromide (0.011 g, 0.104 mmol) and 0.676 g. NaHCO₃ are dissolved in water (12.4 cc) and the aqueous solution is added to the stirred organic fraction at 1000 RPM in the reaction flask. The stirred suspension is cooled to 0°C and the emulsion is re-adjusted to pH=8.4 using 50% solution of HCl. When the temperature of the reactants reached 0°C, 82 g (133 mmol) of 12.1% aqueous NaOCl solution are pumped in via a gas-tight syringe over 90 minutes. During the bleach addition, the pH is maintained at 8.3-8.4 levels using few drops of 50% aqueous HCl. The reaction mixture is stirred for an additional 120 min at 0°C and the organic layer is sampled for GC assay. The reaction in this second stage is kept at pH 8.4 by addition of 0.2-0.25 cc aqueous solution of NaOH (50% concentration). The yield of 3,3-dimethylbutyraldehyde is 89.7% at 60 min and 91.5% at 90 min reaction time.

### EXAMPLE III

Example III represents an oxidation in which the Na₂B₄O₇ co-catalyst is used and the NaHCO₃ is the buffering agent. The example is needed to show that the Na₂B₄O₇ co-catalyst is more efficient then the known KBr based system (compare the results with those from Example II).

16.9 g of 3,3-Dimethyl-1-butanol (117.3 mmol) and 0.0765 g MeO-TEMPO (0.411mmol) are charged in a jacketed glass reaction flask as in Example I. Sodium borate (0.380 g, 1.0 mmol) and 0.676 g NaHCO₃ are dissolved in water (17 cc) and the aqueous solution is added to the stirred organic fraction at 1000 RPM in the reaction flask. The stirred suspension is cooled to 0°C and the emulsion is re-adjusted to pH=8.4 using 50% solution of CH₃COOH. When the temperature of the reactants reached 0°C, 77.5 g (126 mmol) of 12.1% aqueous NaOCl solution are pumped in via a gas-tight syringe over 90 minutes (The pH of the bleach solution was adjusted to 10 using 50% aqueous CH₃COOH). During the bleach addition, the pH was maintained at 8.3-8.4 levels using few drops of 50% aqueous CH₃COOH. The reaction mixture is stirred for an additional 120 min at 0°C and the organic layer is sampled for GC assay. The reaction in this second stage was kept at pH 8.4 by addition of 0.2-0.25 cc aqueous solution of NaOH (50% concentration). The yield of 3,3-dimethylbutyraldehyde is 94.0% at 60 min and 96.0% at 90 min reaction time.

### EXAMPLE IV

Example IV represents an oxidation in which the KBr co-catalyst and the NaHCO₃ buffer were removed and replaced with Na₂B₄O₇ additive.

16.9 g of 3,3-Dimethyl-1-butanol (117.3 mmol) and 0.0765 g MeO-TEMPO (0.411mmol) are charged in a jacketed glass reaction flask as in Example I. Sodium borate (0.330 g, 1.0 mmol) is dissolved in water (12.0 cc) and the aqueous solution is added to the stirred organic fraction at 1000 RPM in the reaction flask. The stirred suspension is cooled to 0°C and the emulsion is re-adjusted to pH=8.4 using 50% solution of CH₃COOH. When the temperature of the reactants reached 0°C, 75.5 g (122 mmol) of 12.1% aqueous NaOCl solution are pumped in via a gas-tight syringe over 90 minutes. During the bleach addition, the pH was maintained at 8.3-8.4 levels using a few drops of 50% aqueous CH₃COOH. The reaction mixture is stirred for an additional 120 min at 0°C and the organic layer is sampled for GC assay. The reaction in this second stage was kept at pH 8.4 by addition of 0.2-0.25 cc aqueous solution of NaOH (50% concentration). The yield of 3,3-dimethylbutyraldehyde is 90.0% at 60 min and 91.0% at 90 min reaction time.

### EXAMPLE V

Example V represents an oxidation in which the KBr co-catalyst and the NaHCO₃ buffer were removed and they were replaced with Na₂B₄O₇-Zr(acetate)₂. additive.

16.9 g of 3,3-Dimethyl-1-butanol (117.3 mmol) and 0.0765 g MeO TEMPO (0.411mmol) are charged in a jacketed glass reaction flask as in Example I. Sodium borate (0.380 g, 1.0 mmol) and Zr(acetate)₂ (0.2 g solution, 0.1 mmol) are dissolved in water (12.0 cc) and the aqueous solution is added to the stirred organic fraction at 1000 RPM in the reaction flask. The stirred suspension is cooled to 0°C and the emulsion is readjusted to pH=8.4 using 50% solution of CH₃COOH. When the temperature of the reactants reached 0°C 75.5 g (122 mmol) of 12.1% aqueous NaOCl solution are pumped in via a gas-tight syringe over 90 minutes (The pH of the bleach solution was adjusted to 10 using 50% aqueous CH₃COOH). During the bleach addition the pH was maintained at 8.3-8.4 levels using few drops of 50% aqueous CH₃COOH. The reaction mixture is stirred for an additional 120 min at 0°C and the organic layer is sampled for GC assay. The reaction in this second stage was kept at pH 8.4 by addition of 0.2-0.25 cc aqueous solution of NaOH (50% concentration). The yield of 3,3-dimethylbutyraldehyde is 95.0% at 60 min and 93.0% at 90 min reaction time.

### EXAMPLE VI

Example VI represents an oxidation in which the KBr co-catalyst and the Na₂B₄O₇ are simultaneously present.

16.9 g of 3,3-Dimethyl-1-butanol (117.3 mmol) and 0.0765 g MeO-TEMPO (0.411mmol) are charged in a jacketed glass reaction flask as in Example I. Sodium borate (0.380 g, 1.0 mmol) and potassium bromide (0.011 g, 0.104 mmol) are dissolved in water (12.0 cc) and the aqueous solution is added to the stirred organic fraction at 1000 RPM in the reaction flask. The stirred suspension is cooled to 0°C and the emulsion is re-adjusted to pH 8.4 using 50% solution of CH₃COOH. When the temperature of the reactants reached 0°C, 75.5 g (122 mmol) of 12.1% aqueous NaOCl solution are pumped in via a gas-tight syringe over 90 minutes (The pH of the bleach solution was adjusted to 10 using 50% aqueous CH₃COOH). During the bleach addition the pH was maintained at 8.3-8.4 levels using a few drops of 50% aqueous CH₃COOH. The reaction mixture is stirred for an additional 120 min at 0°C and the organic layer is sampled for GC assay. The reaction in this second stage was kept at pH 8.4 by addition of 0.2-0.25 cc aqueous solution of NaOH (50% concentration). The yield of 3,3-dimethylbutyraldehyde is 94.0% at 60 min and 95.0% at 90 min reaction time. This yield is equivalent to the yield of Example III in which no potassium bromide was used.

### EXAMPLE VII

Example VII is similar to VI but it exemplifies the robustness of the new catalytic system. Instead of continuously monitoring and maintaining the pH of the emulsion in the second stage, the required amount of NaOH solution was introduced at once at the onset of the second stage of the oxidation. All other concentrations and ratios are the same as in Example III.

16.9 g of 3,3-Dimethyl-1-butanol (117.3 mmol) and 0.0765 g MeO-TEMPO (0.411mmol) are charged in a jacketed glass reaction flask as in Example I. Sodium borate (0.380 g, 1.0 mmol) and 0.676 g NaHCO₃ are dissolved in water (17 cc) and the aqueous solution is added to the stirred organic fraction at 1000 RPM in the reaction flask. The stirred suspension is cooled to 0°C and the emulsion is re-adjusted to pH 8.4 using 50% solution of CH₃COOH. When the temperature of the reactants reached 0°C, 77.5 g (126 mmol) of 12.1% aqueous NaOCl solution are pumped in via a gas-tight syringe over 90 minutes (The pH of the bleach solution was adjusted to 10 using 50% aqueous CH₃COOH). During the bleach addition, the pH was maintained at 8.3-8.4 levels using few drops of 50% aqueous CH₃COOH. The reaction mixture is stirred for an additional 120 min at 0°C and the organic layer is sampled for GC assay. In this Example, 0.2-0.25 cc aqueous solution of NaOH (50% concentration) was added immediately after the bleach addition was completed and no efforts were made to maintain the pH of the emulsion. The yield of 3,3-dimethylbutyraldehyde is 97.0% at 60 min and 94.0% at 90 min reaction time.

### EXAMPLE VIII

Example VIII is similar to VII but the amount of MeO-TEMPO is reduced twice. All other concentrations and ratios are the same as in Example VII.

16.9 g of 3,3-Dimethyl-1-butanol (117.3 mmol) and 0.0382 g MeO-TEMPO (0.205mmol) are charged in a jacketed glass reaction flask as in Example I. Sodium borate (0.380 g, 1.0 mmol) and 0.676 g NaHCO₃ are dissolved in water (17 cc) and the aqueous solution is added to the stirred organic fraction at 1000 RPM in the reaction flask. The stirred suspension is cooled to 0°C and the emulsion is re-adjusted to pH=8.4 using 50% solution of CH₃COOH. When the temperature of the reactants reached 0°C, 77.5 g (126 mmol) of 12.1% aqueous NaOCl solution are pumped in via a gas-tight syringe over 90 minutes (The pH of the bleach solution was adjusted to 10 using 50% aqueous CH₃COOH). During the bleach addition the pH was maintained at 8.3-8.4 levels using few drops of 50% aqueous CH₃COOH. The reaction mixture is stirred for an additional 120 min at 0°C and the organic layer is sampled for GC assay. In this Example, 0.2-0.25 cc aqueous solution of NaOH (50% concentration) was added immediately after the bleach addition was completed and no efforts were made to maintain the pH of the emulsion. The yield of 3,3-dimethylbutyraldehyde is 94.0% at 60 min and 99.0% at 90 min reaction time.

### EXAMPLE IX

Example IX sheds light on the role that the Na₂B₄O₇ plays as an independent co-catalyst to the TEMPO system. In this Example, the sodium borate was completely removed and it was replaced with additional, equimolar amount NaHCO₃. The complete deterioration of the aldehyde yield shows that the presence of Na₂B₄O₇ is crucial in achieving catalyst efficiency under the standard reaction conditions (compare with example VIII).

16.9 g of 3,3-Dimethyl-1-butanol (117.3 mmol) and 0.0382 g MeO-TEMPO (0.205mmol) are charged in a jacketed glass reaction flask as in Example I. NaHCO₃ 0.757 g NaHCO₃ are dissolved in water (17 cc) and the aqueous solution is added to the stirred at 1000 RPM organic fraction in the reaction flask. The stirred suspension is cooled to 0°C and the emulsion is re-adjusted to pH 8.4 using 50% solution of CH₃COOH. When the temperature of the reactants reached 0°C, 77.5 g (126 mmol) of 12.1 % aqueous NaOCl solution are pumped in via a gas-tight syringe over 90 minutes (The pH of the bleach solution was adjusted to 10 using 50% aqueous CH₃COOH). During the bleach addition, the pH was maintained at 8.3-8.4 levels using a few drops of 50% aqueous CH₃COOH. The reaction mixture is stirred for an additional 120 min at 0°C and the organic layer is sampled for GC assay. In this Example, 0.2-0.25 cc aqueous solution of NaOH (50% concentration) was added immediately after the bleach addition was completed and no efforts were made to maintain the pH of the emulsion. The yield of 3,3-dimethylbutyraldehyde is 67.0% at 60 min and 67.0% at 90 min reaction time.

The next series of examples show the use of different 4-substituted TEMPO catalysts and Na₂B₄O₇ co-catalyst.

### EXAMPLE X

12.2 g of 3,3-Dimethyl-1-butanol of 98% purity (117.3 mmol) and 0.043 g MeO-TEMPO (0.223mmol) are charged in a jacketed glass reaction flask as in Example I. Sodium borate (0.294 g, 0.76 mmol), NaHCO₃ (1.472 g, 17.5 mmol) and NaCl (2 g) are dissolved in water (25 cc) and the aqueous solution is added to the stirred organic fraction at 1000 RPM in the reaction flask. The stirred suspension is cooled to 0°C and the emulsion is re-adjusted to pH 8.6 using 40% solution of NaOH. When the temperature of the reactants reached 0°C, 69.8 g (123.1 mmol) of 13.1% aqueous NaOCl solution are pumped in via a gas-tight syringe over 55 minutes. The reaction mixture is stirred for an additional 15 min at 0°C and the organic layer is sampled for GC assay. The yield of 3,3-dimethylbutyraldehyde is 90.6% at 2 min of post bleach-addition time and 92.1 % at 15 min reaction time.

### EXAMPLE XI

12.2 g of 3,3-Dimethyl-1-butanol of 98% purity (117.3 mmol) and 0.036 g TEMPO (0.223mmol) are charged in a jacketed glass reaction flask as in Example I. Sodium borate (0.294 g, 0.76 mmol), NaHCO₃ (1.472 g, 17.5mol) and NaCl (2 g) are dissolved in water (25 cc) and the aqueous solution is added to the stirred organic fraction at 1000 RPM in the reaction flask. The stirred suspension is cooled to 0°C and the emulsion is re-adjusted to pH 8.6 using 40% solution of NaOH. When the temperature of the reactants reached 0°C, 69.8 g (123.1 mmol) of 13.1% aqueous NaOCl solution are pumped in via a gas-tight syringe over 55 minutes. The reaction mixture is stirred for an additional 15 min at 0°C and the organic layer is sampled for GC assay. The yield of 3,3-dimethylbutyraldehyde is 87.4% at 2 min of post bleach-addition time and 91.2% at 15 min reaction time.

### EXAMPLE XII

12.2 g of 3,3-Dimethyl-1-butanol of 98% purity (117.3 mmol) and 0.049 g 4-Acetamido-TEMPO (0.223mmol) are charged in a jacketed glass reaction flask as in Example I. Sodium borate (0.294 g, 0.76 mmol), NaHCO₃ (1.472 g, 17.5mmol) and NaCl (2 g) are dissolved in water (25 cc) and the aqueous solution is added to the stirred organic fraction at 1000RPM in the reaction flask. The stirred suspension is cooled to 0°C and the emulsion is re-adjusted to pH 8.6 using 40% solution of NaOH. When the temperature of the reactants reached 0°C, 69.8 g (123.1 mmol) of 13.1% aqueous NaOCl solution are pumped in via a gas-tight syringe over 55 minutes. The reaction mixture is stirred for an additional 15 min at 0°C and the organic layer is sampled for GC assay. The yield of 3,3-dimethylbutyraldehyde is 88.3% at 2 min of post bleach-addition time and 92.1 % at 15 min reaction time.

### EXAMPLE XIII

8.6 g of heptan-1-ol of 98% purity (72.8 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43 g) are dissolved in water (15 cc) and the aqueous solution is added to the stirred organic fraction at 1000 RPM in the reaction flask. The stirred suspension is cooled to 0°C and the emulsion is re-adjusted to pH=8.6 using 50% solution of CH₃COOH. When the temperature of the reactants reached 0°C, 47.7 g (84.0 mmol) of 13.1% aqueous NaOCl solution are pumped in via a gas-tight syringe over 20 minutes. The reaction mixture is stirred for an additional 15 min at 0°C and the organic layer is sampled for GC assay. The yield of heptanal is 93.0% at 2 min of post bleach-addition time.

### EXAMPLE XIV

7.5 g of hexan-1-ol of 98% purity (72.8 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43 g) are dissolved in water (15 cc) and the aqueous solution is added to the stirred organic fraction at 1000 RPM in the reaction flask. The stirred suspension is cooled to 0°C and the emulsion is re-adjusted to pH 8.6 using 50% solution of CH₃COOH. When the temperature of the reactants reached 0°C, 47.7 g (84.0 mmol) of 13.1% aqueous NaOCl solution are pumped in via a gas-tight syringe over 20 minutes. The reaction mixture is stirred for an additional 15 min at 0°C and the organic layer is sampled for GC assay. The yield of hexanal is 90.0% at 2 min of post bleach-addition time.

### EXAMPLE XV

7.9 g of benzyl alcohol 99% purity (72.3 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43g) are dissolved in water (15 cc) and the aqueous solution is added to the stirred organic fraction at 1000RPM in the reaction flask. The stirred suspension is cooled to 0°C and the emulsion is re-adjusted to pH 8.6 using 50% solution of CH₃COOH. When the temperature of the reactants reached 0°C, 47.7 g (84.0 mmol) of 13.1% aqueous NaOCl solution are pumped in via a gas-tight syringe over 20 minutes. The reaction mixture is stirred for an additional 15 min at 0°C and the organic layer is sampled for GC assay. The yield of benzaldehyde is 100.0% at 2 min of post bleach-addition time.

### EXAMPLE XVI

8.36 g of 4-methylcyclohexanol of 98% purity (71.7 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43 g) are dissolved in water (15 cc) and the aqueous solution is added to the stirred organic fraction at 1000 RPM in the reaction flask. The stirred suspension is cooled to 0°C and the emulsion is re-adjusted to pH 8.4 using 50% solution of CH₃COOH. When the temperature of the reactants reached 0°C, 47.7 g (84.0 mmol) of 13.1% aqueous NaOCl solution are pumped in via a gas-tight syringe over 20 minutes. The reaction mixture is stirred for an additional 5 min at 0°C and the organic layer is sampled for GC assay. The yield of 4-methylcyclohexanone is 94.0% at 5 min of post bleach-addition time.

### EXAMPLE XVII

7.55 g of 4-methy-2-pentanol of 99% purity (73.2 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43 g) are dissolved in water (15 cc) and the aqueous solution is added to the stirred organic fraction at 1000 RPM in the reaction flask. The stirred suspension is cooled to 0°C and the emulsion is re-adjusted to pH 8.4 using 50% solution of CH₃COOH. When the temperature of the reactants reached 0°C, 47.7 g (84.0 mmol) of 13.1% aqueous NaOCl solution are pumped in via a gas-tight syringe over 20 minutes. The reaction mixture is stirred for an additional 5 min at 0°C and the organic layer is sampled for GC assay. The yield of 4-methyl-2-pentanone is 77.0% at 5 min of post bleach-addition time. The same reaction run at 22°C produced the desired 4-methyl-2-pentanon at 92% yield.

### EXAMPLE XVIII

8.68 g of heptanol-1 of 98% purity (73.2 mmol), toluene (10 cc) and 0.047 g MeO-TEMPO (0.252mmol) are charged in a jacketed glass reaction flask as in Example I. Sodium borate (0.129 g, 0.338 mmol), NaHCO₃ (0.43 g) are dissolved in water (15 cc) and the aqueous solution is added to the stirred organic fraction at 1000 RPM in the reaction flask. The stirred suspension is cooled to 0°C and the emulsion is re-adjusted to pH 8.4 using 50% solution of CH₃COOH. When the temperature of the reactants reached 0°C, 95.4 g (168.0 mmol) of 13.1% aqueous NaOCl solution are pumped in via a gas-tight syringe over 40 minutes. The reaction mixture is stirred for an additional 5 min at 0°C and the organic layer is sampled for GC assay. The yield of heptanoic acid is 83.0% at 5 min of post bleach-addition time.

## Claims

1. A process for oxidizing primary and secondary alcohols to aldehydes and ketones, said process comprising reacting the primary or secondary alcohol with an oxidant wherein said alcohol is in a solution including 2,2,6,6,-tetramethylpiperidinyloxy based catalysts selected from the group consisting of catalysts having the formula: 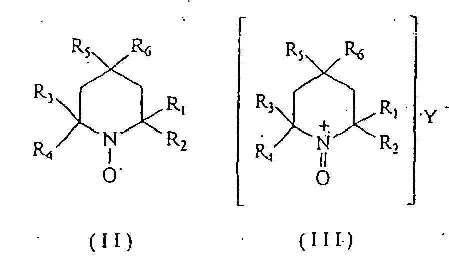 wherein R₁, R₂, R₃, and R₄ are independently lower alkyl with up to 8 carbon atoms or substituted alkyl groups of the same or different structures, R₅ and R₆ are both hydrogen or are lower alkoxy with up to 8 carbon atoms or one is hydrogen and the other is lower alkoxy, hydroxyl, amino, alkyl or dialkylamino, alkylcarbonyloxy, alkylcarbonylamino, or can be jointly substituted by an oxygen or a ketal, and the Y⁻ is an anion,
and further in the presence of a Na₂B₄O₇ co-catalyst, said alcohol acting as the substrate in said solution.

2. A process according to claim 1, wherein said primary and second alcohols are selected from the group consisting of methanol, ethanol, n- and isopropyl alcohol, n-, iso- and sec-butyl alcohol, pentyl alcohol, hexyl alcohol, neopentyl alcohol, neohexyl alcohol, octyl alcohol, lauryl alcohol, tridecyl alcohol, myristyl alcohol, nonadecyl alcohol, eicosyl alcohol, unsaturated alcohols including but not limited to allyl alcohol, crotyl alcohol, and propargyl alcohol, and aromatic alcohols including but not limited to benzyl alcohol, phenyl ethanol, and phenyl propanol.

3. A process according to claim 1 or 2, wherein the 2,2,6,6-tetrmethylpiperidinyloxy based catalyst is present in an amount of 0.001-10 mol% of the substrate alcohol.

4. A process according to any one of the preceding claims, wherein said TEMPO based catalyst is selected from the group consisting of 4-methoxy-TEMPO, 4-ethoxy-TEMPO, 4-acetoxy-TEMPO, 4-acetamino-TEMPO, 4-hydroxy-TEMPO, 4-benzoyloxy-TEMPO, 4-amino-TEMPO, N, N-dimethylamino-TEMPO, 4-oxo-TEMPO, poly [(6-[1,1,3,3-tetramethylbutyl)amino]-s-triazine-2,4-diyl], [(2,2,6,6-tetramyetho-4-Piperidyl)imino] hexamethylene[)2,2,6,6-tetramethyl-4-piperidnyl)imino]] and a combination of these.

5. A process according to any one of the preceding claims wherein the co-catalyst concentration is 0.01-20% mol of the substrate alcohol.

6. A process according to any one of the preceding claims, wherein the oxidant is selected from the group consisting of sodium hypochlorite, potassium hypochlorite, calcium hypochlorite, sodium chlorite, hydrogen peroxide, tert-butyl hydroperoxide, trichloroisocyanuric acid, peracetic acid, performic acid, trichloroperacetic acid and trifluoroperacetic acid.

7. A process according to claim 6, wherein the ratio between the oxidant and the alcohol is in the range of from 1:0.8 to 1:1.5.

8. A process according to any one of the preceding claims, further comprising the addition of one or more additional solvents to the alcohol solution.

9. A process according to claim 8, wherein said solvent is selected from the group consisting of water, acetonitrile, acetone, tetrahydrofuran, benzene, toluene, methyl tert-butyl ether, methylene chloride, chloroform, carbon tetrachloride, pentane, hexane, heptane, ethylacetate, methyl acetate and a mixture of solvents from the group above.

10. A process according to any one of claim 1 to 7, wherein the oxidation is carried out in absence of an additional solvent.

11. A process according to any one of the preceding claims, wherein a buffer solution is added to the alcohol solution.

12. A process according to claim 11, wherein said buffer solution comprises a solution selected from the group consisting of NaHCO₃, KHCO₃, Na₂CO₃, K2CO₃, K₂PO₄, Na₂HPO₄, NaH₂PO₄, K₂HPO₄, KH₂PO₄, NaOAc and any combination thereof sufficient to maintain a pH of from 4 to 12.

13. A process according to any one of the preceding claims, wherein the reaction temperature is maintained in the range of -10°C to 50°C.

14. A process according to any one of claims 11 to 13, wherein a bleach solution is added to the solution of alcohol and aqueous buffer while maintaining the pH of the emulsion in the range of pH 4-12.

15. A process according to claim 14, wherein the addition time for the bleach solution is between completion addition at one time and 10 hours and the post addition reaction is continued for an additional 0 to 10 hours.

16. A process according to claim 15, wherein a solution of the catalyst in the alcohol substrate is added to the buffered bleach solution, containing the co-catalyst, over an extended period of time while maintaining the pH of the emulsion in the range of pH 4-12.

17. A process according to claim 16, wherein the addition time for the catalyst-alcohol solution is between complete addition at one time and 10 hours and the post addition reaction is continued for an additional 0 to 30 hours.

18. A method according to any one of the preceding claims, comprising the additional step of purification of the crude aldehyde or ketone via distillation, fractional distillation, either batch or continuous or a thin-film evaporator.

19. A process of claim 1, wherein the alcohol is 3,3-dimethylbutanol and wherein said 3,3-dimethylbutanol is oxidized to 3,3-dimethylbutyraldehyde.

20. A process according to any one of the preceding claims, wherein two or more catalysts each selected from the group consisting of 2,2,6,6,-tetramethylpiperidinyloxy based catalysts are included in said alcohol solution.

## Patentansprüche

1. Verfahren zum Oxidieren von primären und sekundären Alkoholen zu Aldehyden und Ketonen, wobei das Verfahren das Umsetzen des primären oder sekundären Alkohols mit einem Mittel zum Oxidieren umfasst und wobei sich der Alkohol in einer Lösung befindet, die auf 2,2,6,6-Tetramethylpiperidinyloxy basierende Katalysatoren einschließt, die ausgewählt sind aus der Gruppe, bestehend aus Katalysatoren, welche die Formel haben: 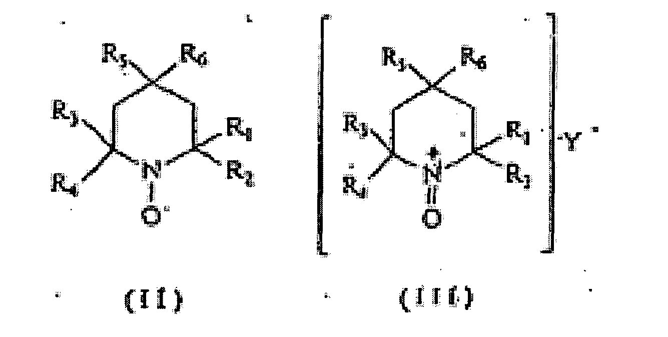 worin R₁, R₂, R₃ und R₄ unabhängig niederes Alkyl mit bis zu 8 Kohlenstoffatomen oder substituierte Alkyl-Gruppen mit den gleichen oder verschiedenen Strukturen sind, R₅ und R₆ sind beide Wasserstoff oder sind niederes Alkoxy mit bis zu 8 Kohlenstoffatomen oder das eine ist Wasserstoff und das andere ist niederes Alkoxy, Hydroxyl, Amino, Alkyl oder Dialkylamino, Alkylcarbonyloxy, Alkylcarbonylamino, oder können gemeinsam substituiert sein durch einen Sauerstoff oder ein Ketal, und das Y ist ein Anion;
und ferner in Gegenwart eines Na₂B₄O₇-Cokatalysators, wobei der Alkohol als das Substrat in dieser Lösung wirkt.

2. Verfahren nach Anspruch 1, bei welchem die primären und sekundären Alkohole ausgewählt sind aus der Gruppe, bestehend aus Methanol, Ethanol, n-und Isopropanol, n-, Iso- und sec-Butylalkohol, Pentylalkohol, Hexylalkohol, Neopentylalkohol, Neohexylalkohol, Octylalkohol, Laurylalkohol, Tridecylalkohol, Myristylalkohol, Nonadecylalkohol, Eikosylalkohol, ungesättigten Alkoholen, die die folgenden einschließen, ohne auf diese beschränkt zu sein: Allylalkohol, Crotylalkohol und Propargylalkohol; sowie aromatischen Alkoholen, die die folgenden einschließen, ohne auf diese beschränkt zu sein: Benzylalkohol, Phenylethanol und Phenylpropanol.

3. Verfahren nach Anspruch 1 oder 2, bei welchem der auf 2,2,6,6-Tetramethylpiperidinyloxy basierende Katalysator in einer Menge von 0,001% bis 10 Molprozent des Substratalkohols vorliegt.

4. Verfahren nach einem der vorgenannten Ansprüche, wobei der auf TEMPO basierende Katalysator ausgewählt ist aus der Gruppe, bestehend aus 4-Methoxy-TEMPO, 4-Ethoxy-TEMPO, 4-Acetoxy-TEMPO, 4-Acetamino-TEMPO, 4-Hydroxy-TEMPO, 4-Benzyloxy-TEMPO, 4-Amino-TEMPO, 4-N,N-Dimethylamino-TEMPO, 4-Oxo-TEMPO, Poly[(6-[1,1,3,3-tetramethylbutyl)amino]-s-triazin-2,4-diyl], [(2,2,6,6-Tetramyetho-4-piperidinyl)imino]hexamethylen[)2,2,6,6-tetramethyl-4-piperidinyl)imino]], sowie einer Kombination von diesen.

5. Verfahren nach einem der vorgenannten Ansprüche, bei welchem die Konzentration des Cokatalysators 0,01 % bis 20 Molprozent des Substratalkohols beträgt.

6. Verfahren nach einem der vorgenannten Ansprüche, bei welchem das Mittel zum Oxidieren ausgewählt ist aus der Gruppe, bestehend aus Natriumhypochlorit, Kaliumhypochlorit, Calciumhypochlorit, Natriumchlorit, Wasserstoffperoxid, *tert*-Butylhydroperoxid, Trichlorisocyanursäure, Peressigsäure, Perameisensäure, Trichlorperessigsäure sowie Trifluorperessigsäure.

7. Verfahren nach Anspruch 6, bei welchem das Verhältnis zwischen dem Mittel zum Oxidieren und dem Alkohol im Bereich von 1:0,8 bis 1:1,5 liegt.

8. Verfahren nach einem der vorgenannten Ansprüche ferner umfassend die Zugabe von einem oder mehren zusätzlichen Lösemitteln zu der Alkohollösung.

9. Verfahren nach Anspruch 8, wobei das Lösemittel ausgewählt ist aus der Gruppe, bestehend aus Wasser, Acetonitril, Aceton, Tetrahydrofuran, Benzol, Toluol, Methyl-*tert*-butylether, Methylenchlorid, Chloroform, Tetrachlorkohlenstoff, Pentan, Hexan, Heptan, Ethylacetat, Methylacetat und einer Mischung von Lösemitteln aus der vorgenannten Gruppe.

10. Verfahren nach einem der Ansprüche 1 bis 7, bei welchem die Oxidation in Abwesenheit eines zusätzlichen Lösemittels ausgeführt wird.

11. Verfahren nach einem der vorgenannten Ansprüche, bei welchem der Lösung des Alkohols eine Pufferlösung zugegeben wird.

12. Verfahren nach Anspruch 11, bei welchem die Pufferlösung eine Lösung umfaßt, die ausgewählt ist aus der Gruppe, bestehend aus: NaHCO₃, KHCO₃, Na₂CO₃, K₂CO₃, K₂PO₄, Na₂HPO₄, NaH₂PO₄, K₂HPO₄, KH₂PO₄, NaOAc sowie beliebige Kombinationen von diesen, die ausreichend sind, um einen pH-Wert von 4 bis 12 aufrecht zu erhalten.

13. Verfahren nach einem der vorgenannten Ansprüche, bei welchem die Reaktionstemperatur im Bereich von -10° bis 50°C gehalten wird.

14. Verfahren nach einem der Ansprüche 11 bis 13, bei welchem der Lösung des Alkohols und wässrigen Puffers eine Bleichlösung zugegeben wird, während der pH-Wert der Emulsion im Bereich von pH 4 bis 12 gehalten wird.

15. Verfahren nach Anspruch 14, bei welchem die Dauer für die Zugabe der Bleichlösung zwischen der vollständigen Zugabe der Bleichlösung mit einem Mal und zehn Stunden liegt und die Reaktion nach der Zugabe für weitere Null bis zehn Stunden fortgesetzt wird.

16. Verfahren nach Anspruch 15, bei welchem eine Lösung des Katalysators in dem Alkoholsubstrat zu der gepufferten Bleichlösung, die den Cokatalysator enthält, über eine verlängerte Zeitdauer zugegeben wird, während der pH-Wert der Emulsion im Bereich von pH 4 bis 12 gehalten wird.

17. Verfahren nach Anspruch 16, bei welchem die Dauer der Zugabe für die Lösung von Alkohol und Katalysator zwischen der vollständigen Zugabe auf einem Mal und 10 Stunden liegt und die Reaktion nach der Zugabe für weitere Null bis 30 Stunden fortgesetzt wird.

18. Verfahren nach einem der vorgenannten Ansprüche, umfassend den weiteren Schritt der Reinigung des rohen Aldehyds oder Ketons auf dem Wege einer Destillation, fraktionierten Destillation, entweder diskontinuierlich oder kontinuierlich, oder eines Dünnschichtverdampfers.

19. Verfahren nach Anspruch 1, bei welchem der Alkohol 3,3-Dimethylbutanol ist und wobei das 3,3-Dimethylbutanol zu 3,3-Dimethylbutyraldehyd oxidiert ist.

20. Verfahren nach einem der vorgenannten Ansprüche, bei welchem zwei oder mehrere Katalysatoren, die ausgewählt sind aus der Gruppe, bestehend aus auf 2,2,6,6-Tetramethylpiperidinyloxy basierenden Katalysatoren, in die genannte Alkohollösung einbezogen sind.

## Revendications

1. Procédé d'oxydation d'alcools primaires et secondaires en aldéhydes et en cétones, ledit procédé comprenant la réaction de l'alcool primaire ou secondaire avec un oxydant, dans lequel ledit alcool est dans une solution comprenant des catalyseurs à base de 2,2,6,6-tétraméthylpipéridinyloxy choisis dans le groupe constitué de catalyseurs présentant la formule : 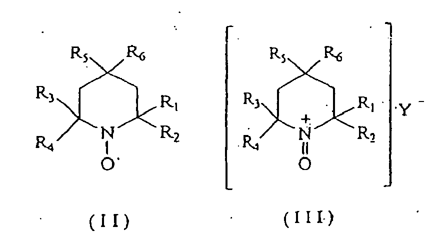 dans lesquelles R₁, R₂, R₃ et R₄ sont indépendamment un groupe alkyle inférieur ayant jusqu'à 8 atomes de carbone ou des groupes alkyle substitués de structures identiques ou différentes, R₅ et R₆ sont tous deux l'hydrogène ou sont un groupe alcoxy inférieur avec jusqu'à 8 atomes de carbone ou l'un est l'hydrogène et l'autre est un groupe alcoxy inférieur, hydroxyle, amino, alkyle ou dialkylamino, alkylcarbonyloxy, alkylcarbonylamino ou peuvent être substitués ensemble par un oxygène ou un groupe cétal, et Y⁻ est un anion,
et de plus en présence d'un co-catalyseur de Na₂B₄O₇, ledit alcool agissant comme le substrat dans ladite solution.

2. Procédé selon la revendication 1, dans lequel lesdits alcools primaires et secondaires sont choisis dans le groupe constitué du méthanol, de l'éthanol, de l'alcool n- et isopropylique, de l'alcool n-, iso- et sec-butylique, de l'alcool pentylique, de l'alcool hexylique, de l'alcool néopentylique, de l'alcool néohexylique, de l'alcool octylique, de l'alcool laurylique, de l'alcool tridécylique, de l'alcool myristilique, de l'alcool nonadécylique, de l'alcool eicosylique, d'alcools insaturés incluant l'alcool allylique, l'alcool crotylique et l'alcool propargylique mais n'étant pas limités à ceux-ci, et d'alcools aromatiques comprenant l'alcool benzylique, le phényléthanol et le phénylpropanol mais n'étant pas limités à ceux-ci.

3. Procédé selon la revendication 1 ou 2, dans lequel le catalyseur à base de 2,2,6,6-tétraméthylpipéridinyloxy est présent dans une quantité de 0,001-10 % en mol de l'alcool de substrat.

4. Procédé selon l'une quelconque des revendications précédentes, dans lequel ledit catalyseur à base de TEMPO est choisi dans le groupe constitué de 4-méthoxy-TEMPO, 4-éthoxy-TEMPO, 4-acétoxy-TEMPO, 4-acétamino-TEMPO, 4-hydroxy-TEMPO, 4-benzoyloxy-TEMPO, 4-amino-TEMPO, N,N-diméthylamino-TEMPO, 4-oxo-TEMPO, poly[[6-[(1,1,3,3-tétraméthylbutyl)amino]-s-triazine-2,4-diyl], [(2,2,6,6-tétraméthyl-4-pipéridyl)imino]hexaméthylène[(2,2,6,6-tétraméthyl-4-pipéridinyl)imino]] et une combinaison de ceux-ci.

5. Procédé selon l'une quelconque des revendications précédentes, dans lequel la concentration en co-catalyseur est de 0,01-20 % en mol de l'alcool de substrat.

6. Procédé selon l'une quelconque des revendications précédentes, dans lequel l'oxydant est choisi dans le groupe constitué de l'hypochlorite de sodium, de l'hypochlorite de potassium, de l'hypochlorite de calcium, du chlorite de sodium, du peroxyde d'hydrogène, de l'hydroperoxyde de tert-butyle, de l'acide trichloroisocyanurique, de l'acide peracétique, de l'acide performique, de l'acide trichloroperacétique et de l'acide trifluoroperacétique.

7. Procédé selon la revendication 6, dans lequel le rapport entre l'oxydant et l'alcool se trouve dans l'intervalle de 1:0,8 à 1:1,5.

8. Procédé selon l'une quelconque des revendications précédentes comprenant de plus l'addition d'un ou plusieurs solvants supplémentaires à la solution d'alcool.

9. Procédé selon la revendication 8, dans lequel ledit solvant est choisi dans le groupe constitué de l'eau, de l'acétonitrile, de l'acétone, du tétrahydrofurane, du benzène, du toluène, du méthyl-tert-butyléther, du chlorure de méthylène, du chloroforme, du tétrachlorure de carbone, du pentane, de l'hexane, de l'heptane, de l'acétate d'éthyle, de l'acétate de méthyle et d'un mélange de solvants du groupe ci-dessus.

10. Procédé selon l'une quelconque des revendications 1 à 7, dans lequel l'oxydation est réalisée en l'absence d'un solvant supplémentaire.

11. Procédé selon l'une quelconque des revendications précédentes, dans lequel une solution tampon est ajoutée à la solution d'alcool.

12. Procédé selon la revendication 11, dans lequel ladite solution tampon comprend une solution choisie dans le groupe constitué de NaHCO₃, KHCO₃, Na₂CO₃, K₂CO₃, K₂PO₄, Na₂HPO₄, NaH₂PO₄, K₂HPO₄, KH₂PO₄, NaOAc et d'une combinaison quelconque de ceux-ci suffisante pour maintenir un pH de 4 à 12.

13. Procédé selon l'une quelconque des revendications précédentes, dans lequel la température de la réaction est maintenue dans l'intervalle de -10°C à 50°C.

14. Procédé selon l'une quelconque des revendications 11 à 13, dans lequel une solution de blanchiment est ajoutée à la solution d'alcool et au tampon aqueux tout en maintenant le pH de l'émulsion dans l'intervalle de pH 4-12.

15. Procédé selon la revendication 14, dans lequel la durée d'addition pour la solution de blanchiment est comprise entre l'addition complète en une fois et 10 h et la réaction de post-addition est prolongée pendant de 0 à 10 h supplémentaires.

16. Procédé selon la revendication 15, dans lequel une solution du catalyseur dans le substrat d'alcool est ajoutée à la solution de blanchiment tamponnée contenant le co-catalyseur sur une durée prolongée tout en maintenant le pH de l'émulsion dans l'intervalle de pH 4-12.

17. Procédé selon la revendication 16, dans lequel la durée d'addition pour la solution de catalyseur-alcool est comprise entre l'addition complète en une fois et 10 h et la réaction de post-addition est prolongée pendant de 0 à 30 h supplémentaires.

18. Procédé selon l'une quelconque des revendications précédentes comprenant l'étape supplémentaire de purification de l'aldéhyde ou de la cétone brute via une distillation, une distillation fractionnée, soit discontinue, soit continue ou un évaporateur à couche mince.

19. Procédé selon la revendication 1, dans lequel l'alcool est le 3,3-diméthylbutanol et dans lequel ledit 3,3-diméthylbutanol est oxydé en 3,3-diméthylbutyraldéhyde.

20. Procédé selon l'une quelconque des revendications précédentes, dans lequel deux ou plusieurs catalyseurs choisis chacun dans le groupe constitué de catalyseurs à base de 2,2,6,6-tétraméthylpipéridinyloxy sont inclus dans ladite solution d'alcool.
